# EUROPÄISCHE PATENTSCHRIFT

(11) **EP 3 031 439 B1**
(45) Veröffentlichungstag und Bekanntmachung des Hinweises auf die Patenterteilung: **25.08.2021**
(21) Anmeldenummer: 15196960.7
(22) Anmeldetag: 03.03.2009
(51) Int. Cl.: A61H 23/02, B06B 1/02, A61N 7/00, A61N 1/32, A61B 17/30, A61N 5/06

(54) **SYSTEM ZUR ERZEUGUNG VON ULTRASCHALLWELLEN**
SYSTEM FOR GENERATING ULTRASONIC WAVES
SYSTEME DE GENERATION D'ONDES A ULTRASONS

(30) Priorität: 03.03.2008 WO PCT/EP2008/001675
(43) Veröffentlichungstag der Anmeldung: 15.06.2016
(62) Teilanmeldung aus: 09719522.6
(73) Patentinhaber: Wellcomet GmbH, 76137 Karlsruhe (DE)
(72) Erfinder: Kruglikov, Ilja, 76351 Linkenheim-Hochstetten (DE)
(74) Vertreter: Lemcke, Brommer & Partner Patentanwälte Partnerschaft mbB

(56) Entgegenhaltungen:
- EP-A- 1 747 817
- EP-A- 1 747 818
- EP-A1- 1 785 164
- EP-A1- 1 844 750
- EP-A2- 0 612 570
- WO-A2-2004/080147
- US-A1- 2007 038 094

## Beschreibung

Die Erfindung betrifft ein System zur Erzeugung von Ultraschallwellen gemäß dem Oberbegriff des Anspruchs 1.

Ultraschallwellen werden zur Beaufschlagung von biologischem Gewebe für medizinische, therapeutische oder kosmetische Anwendungen, sowie für Forschungszwecke benutzt. Üblicherweise werden mit dem Begriff Ultraschallwellen Schwingungen mit einer Frequenz größer 20 kHz bezeichnet. Häufig ist hierbei die Verwendung von niederfrequenten Ultraschallfrequenzen im Bereich von 20 kHz bis 100 kHz und die Verwendung von hochfrequenten Ultraschallfrequenzen über 0,8 MHz.

Systeme zur Erzeugung von Ultraschallwellen für die Behandlung von biologischem Gewebe weisen üblicherweise einen Signalgenerator auf, welcher zur Erzeugung von periodischen elektrischen Signalen dient. Ferner umfassen typische Systeme einen Ultraschallkopf, welcher mittels einer elektrischen Leitung mit einem Signalausgang des Signalgenerators verbunden ist. Der Ultraschallkopf besitzt einen Bereich zur Auflage auf das biologische Gewebe, wobei im Ultraschallkopf in der Nähe dieses Auflagebereiches die vom Signalgenerator erzeugten periodischen elektrischen Signale in Ultraschallwellen umgewandelt werden.

Typischerweise ist ein Ultraschallkopf auf die Umwandlung von elektrischen Signalen mit einer festen Frequenz ausgelegt. Es sind jedoch auch Ultraschallköpfe bekannt, welche elektrische Signale mit unterschiedlichen Frequenzen in entsprechende Ultraschallwellen umwandeln können:
Die US 5,460,595 beschreibt ein System zur Erzeugung von Ultraschallwellen, bei dem der Signalgenerator und der Ultraschallkopf derart ausgelegt sind, dass Ultraschallwellen mit drei verschiedenen Frequenzen erzeugt werden können, ohne dass der Ultraschallkopf ausgetauscht werden müsste.

Dieses System stellt dem Anwender drei verschiedene Ultraschallfrequenzen zur Verfügung, um drei verschiedene Eindringtiefen in das biologische Gewebe zu bewirken.

Wie tief und mit welcher Intensität der Ultraschall in den Körper eindringt, hängt von der Frequenz der Ultraschallwelle ab. Ein Maß für die Eindringtiefe ist die so genannte Halbwertstiefe, das heißt die Strecke, nach der sich die Schallintensität in dem biologischen Gewebe auf 50 % durch Absorption vermindert hat. Je geringer die Frequenz, desto höher die Eindringtiefe. Für typisches biologisches Gewebe eines Menschen beträgt die Halbwertstiefe einer Ultraschallwelle mit Frequenz 3 MHz etwa 1 cm und für eine Ultraschallwelle mit der Frequenz 1 MHz etwa 3 cm.

Die Halbwertstiefe ist unabhängig von der eingestrahlten Intensität, wohingegen die bis zu einer gewissen Tiefe in dem biologischen Gewebe absorbierte Energie sowohl von der Frequenz als auch von der Intensität der Ultraschallstrahlung abhängt.

Mit dem bekannten System lassen sich somit die Halbwertstiefe mittels der benutzten Ultraschallfrequenz und die bis zu einer bestimmten Tiefe absorbierte Energie mittels der gewählten Intensität variieren. Wünschenswert wäre es jedoch, bei der Ultraschallbehandlung die Massagewirkung, das heißt die Relativbewegung einzelner Teile des behandelten biologischen Gewebes stärker beeinflussen zu können.

Ein System mit den Merkmalen des Oberbegriffes des Anspruches 1 ist durch die EP 1 747 818 offenbart. Weitere Systeme zur Erzeugung von Ultraschallwellen sind durch die US 6148225, EP 1 262 160 und US 5178134 bekannt geworden. Dort wird jeweils die Behandlungsfrequenz ständig geändert.

Der Erfindung liegt die Aufgabe zugrunde, ein System und ein Verfahren zur Erzeugung von Ultraschallwellen zu schaffen, welches die Möglichkeiten der Beeinflussung von biologischem Gewebe durch Ultraschallwellen erweitert, insbesondere neue Massagewirkungen ermöglicht.

Diese Aufgabe wird gemäß Anspruch 1 dadurch gelöst, dass zumindest während einer vorgegebenen Zeitspanne zwischen zumindest zwei vorgegebenen Frequenzen *f₁* und *f₂* umgeschaltet wird, wobei nach Umschalten auf die Frequenz *f₁* Ultraschallwellen mit dieser Frequenz für eine Zeitspanne *T₁* erzeugt werden und nach Umschalten auf die Frequenz *f₂* Ultraschallwellen mit dieser Frequenz für eine zu *T₁* unterschiedliche Zeitspanne *T₂* erzeugt werden und wobei die der höheren Frequenz zugeordnete Zeitspanne größer ist als die der niedrigeren Frequenz zugeordnete Zeitspanne. Ist somit *f₁* größer als *f₂*, so ist auch *T₁* größer als *T₂* und umgekehrt.

Dabei geht die Erfindung von folgender Erkenntnis aus:
Die Ultraschallbeaufschlagung führt in dem biologischen Gewebe abwechselnd zu Bereichen mit Überdruck und Bereichen mit Unterdruck, wobei der Druckwert, das heißt insbesondere die Differenz des Drucks zwischen den Bereichen mit Überdruck und den Bereichen mit Unterdruck frequenzunabhängig ist.

Frequenzabhängig ist jedoch die Entfernung der Bereiche mit Überdruck und der Bereiche mit Unterdruck. Zwei Bereiche mit Überdruck sind in etwa um eine Strecke voneinander entfernt, welche der Wellenlänge der verwendeten Ultraschallstrahlung entspricht. Etwa mittig zwischen diesen Bereichen mit Überdruck, das heißt in etwa eine halbe Wellenlänge entfernt, befindet sich ein Bereich mit Unterdruck. Je höher die Frequenz, desto geringer die Wellenlänge und damit der Abstand zwischen Bereichen mit Über- und Unterdruck. Auch wenn sich die Höhe des Drucks mit der Frequenz der verwendeten Ultraschallstrahlung nicht ändert, so ändert sich jedoch der Abstand zwischen Bereichen mit Über- und Unterdruck und damit der Druckgradient im Gewebe. Nach Untersuchungen der Anmelderin ist die Geschwindigkeit, mit der zwischen den mindestens zwei Ultraschall-Frequenzen gewechselt wird, also die Umschaltfrequenz, ganz wesentlich für den therapeutischen Effekt der Behandlung. Die Umschaltfrequenz gibt an, wie oft pro Sekunde ein Frequenzwechsel stattfindet, d. h. von der aktuellen Ultraschallfrequenz zu der nächsten Ultraschall-Frequenz übergegangen wird.

Dabei hat sich herausgestellt, dass die erfindungsgemäßen Umschaltfrequenzen in einer optimalen Relation zur Eigenelastizität der Gewebezellen, insbesondere der Zellmembranen stehen. So wurde durch Anwendung der genannten Umschaltfrequenzen eine bedeutend intensivere Einwirkung auf die Zelle registriert mit einer Steigerung der Durchlässigkeit der Zellmembranen bis auf 200 %. Diese Verbesserung der Zell-Diffusion führt zu einer deutlichen Straffung des Gewebes, was insbesondere bei Cellulites von Vorteil ist, aber auch zur Behandlung von Arthrose und Arthritis eingesetzt werden kann.

Daneben ist auch die Wahl der Ultraschall-Frequenzen von Bedeutung. Vorzugsweise wird mit Frequenzen von 0,7 MHz bis etwa 10 MHz gearbeitet, wobei die geringeren Frequenzen für eine hohe Eindringtiefe von einigen Zentimetern in das Gewebe, die hohen Frequenzen für eine eher oberflächliche Gewebebehandlung mit einer Eindringtiefe von weniger als 1 Zentimeter empfehlenswert sind.

Eine wesentliche Erkenntnis hierbei ist, dass über die Frequenzwahl nicht nur die Eindringtiefe vorgegeben wird, sondern für unterschiedliche Frequenzen auch eine unterschiedliche Beanspruchung des Gewebes erfolgt. Es ist daher wünschenswert, nicht nur mit einer ausreichenden Geschwindigkeit zwischen den Frequenzen hin- und herzuschalten, sondern darüber hinaus für unterschiedliche Frequenzen unterschiedliche Zeitspannen vorzugeben, sodass das Gewebe mit unterschiedlichen Frequenzen beaufschlagt wird und für jede Frequenz eine spezifische Zeitspanne vorgebbar ist.

Hierdurch wird eine weitere Optimierung des Behandlungsvorgangs erreicht, denn für jede Frequenz kann die jeweils optimale Zeitspanne vorgegeben werden.

Das erfindungsgemäße System zur Erzeugung von Ultraschallwellen ist daher derart ausgeführt, dass nach Umschalten auf die Frequenz *f₁* für eine vorgegebene Zeitspanne *T₁* Ultraschallwellen mit dieser Frequenz erzeugt wird und ebenso nach Umschalten auf die Frequenz *f₂* Ultraschallwellen für eine vorgegebene Zeitspanne *T₂* mit der Frequenz *f₂* erzeugt werden, wobei *T₁* und *T₂* unterschiedlich sind.

Hierbei ist die der höheren Frequenz zugeordnete Zeitspanne größer als die der niedrigeren Frequenz zugeordnete Zeitspanne. Dies ist in der Erkenntnis der Anmelderin begründet, dass unerwünschte Nebeneffekte typischerweise in größerem Maße bei niedrigeren Frequenzen auftreten. Geringere Frequenzen weisen eine größere Eindringtiefe auf und dadurch besteht beispielsweise eine höhere Gefahr, dass ein unter dem zu behandelnden Gewebe liegender Knochen ebenfalls einen Energieeintrag erfährt, der zu unerwünschten Nebeneffekten führt, wie beispielsweise Knochenhautschmerzen oder eine Knochenhautentzündung. Daher wird bei dem erfindungsgemäßen Verfahren der geringeren Frequenz eine geringere Zeitdauer zugeordnet, so dass die geringere Frequenz mit einer kürzeren Zeitdauer kontinuierlich angewandt wird.

Sofern das erfindungsgemäße System lediglich zur Erzeugung zweier Frequenzen *f₁* und *f₂* ausgebildet ist, wird während der vorgegebenen Behandlungszeit somit zwischen den Frequenzen *f₁* und *f₂* hin- und hergeschaltet, wobei nach jedem Umschaltvorgang die jeweilige Frequenz für eine für diese Frequenz spezifisch vorgegebene Zeitspanne erzeugt wird und entsprechend das Gewebe für diese Zeitspanne mit Ultraschallwellen mit der erzeugten Frequenz beaufschlagt wird.

Erfindungsgemäß liegen die Zeitspannen T₁ und T₂ im Bereich 0,5 ms (Millisekunden) bis 50 ms. Untersuchungen der Anmelderin haben gezeigt, dass vorteilhafterweise die Zeitspannen *T₁* und *T₂* im Bereich 1 ms bis 20 ms liegen. Hierdurch ist ein ausreichend schneller Frequenzwechsel gewährleistet.

Wird eine Ultraschallbehandlung über eine längere Zeitdauer mit einer konstanten Frequenz statisch durchgeführt, so bildet sich in dem Gewebe eine stehende Welle aus. Dies kann zur Ausbildung so genannter "Hot Spots" führen, d. h. lokalen Bereichen mit hohem Energieeintrag und Wärmeentwicklung. Diese Hot Spots können unter Anderem Knochenhautschmerzen oder Kochenhautentzündungen zur Folge haben. Daher ist es vorteilhaft, wie zuvor angegeben, zum Einen unterschiedliche Frequenzen zu verwenden und zwischen diesen schnell umzuschalten, so dass eine Frequenz nur für eine kurze Zeitdauer vorzugsweise in den zuvor angegebenen Bereichen für die Zeitspannen *T*₁ und *T*₂ angewendet werden. Insbesondere sind Zeitspannen kleiner gleich 10 ms, vorzugsweise kleiner gleich 2 ms vorteilhaft.

Durch die zuvor beschriebene Ausführungsform und die Vermeidung von Hot Spots ist es insbesondere möglich, eine Behandlung ohne Bewegung des Ultraschallkopfes durchzuführen. Bei dem im Stand der Technik bekannten Vorrichtungen muss typischerweise der Ultraschallkopf hin- und herbewegt werden, um eine zu hohe Belastung insbesondere des Knochens oder der Knochenhaut zu vermeiden.

Weiterhin ist es vorteilhaft, wenn die Zeitspannen *T₁* und *T*₂ ein Verhältnis *T₁*/*T₂* von etwa 3/7 oder etwa 1/3 oder etwa 1/4 aufweisen, sofern *f₁* kleiner als *f₂* ist bzw. die entsprechenden Reziprokwerte, sofern *f₁* größer als *f₂* ist.

Vorzugsweise ist für die Frequenz *f₁* eine Zeitspanne *T₁* von 1 ms vorgegeben wird und für Frequenz *f₂* eine Zeitspanne *T*₂ von 4 ms vorgegeben oder für die Frequenz *f₁* eine Zeitspanne *T₁* von 3 ms vorgegeben und für Frequenz *f₂* eine Zeitspanne *T*₂ von 7 ms oder für die Frequenz *f₁* eine Zeitspanne *T₁* von 5 ms vorgegeben und für Frequenz *f₂* eine Zeitspanne *T*₂ von 15 ms.

Hierbei ist es insbesondere vorteilhaft, die Frequenz *f₁* mit etwa 1 MHz zu wählen und die Frequenz *f₂* mit etwa 3 MHz zu wählen oder die Frequenz *f₁* mit etwa 3 MHz zu wählen und die Frequenz *f₂* mit etwa 10 MHz.

Untersuchungen der Anmelderin haben ergeben, dass insbesondere die nachfolgend aufgelisteten Kombinationen von Frequenzen und Zeitspannen für die Gewebebehandlung vorteilhaft sind:

| *f₁* | *f₂* | *T₁* | *T*₂ |
|---|---|---|---|
| 1 MHz | 3 MHz | 1 ms | 4 ms |
| 1 MHz | 3 MHz | 3 ms | 7 ms |
| 1 MHz | 3 MHz | 5 ms | 15 ms |
| 3 MHz | 10 MHz | 1 ms | 4 ms |
| 3 MHz | 10 MHz | 3 ms | 7 ms |
| 3 MHz | 10 MHz | 5 ms | 15 ms |

Ebenso liegt es im Rahmen der Erfindung, mehr als zwei unterschiedliche Frequenzen zu erzeugen und entsprechend zwischen mehr als zwei unterschiedlichen Frequenzen in einer vorgegebenen Abfolge umzuschalten. Hierbei wird für jede der vorgegebenen Frequenzen jeweils eine dieser Frequenz zugeordneten Zeitspanne vorgegeben und bei dem Umschaltvorgang werden nach dem Umschalten auf eine Frequenz Ultraschallwellen dieser Frequenz für die zu dieser Frequenz vorgegebenen Zeitspanne erzeugt, worauf der Umschaltvorgang auf die nächste Frequenz gemäß der vorgegebenen Abfolge vorgenommen wird.

Vorteilhafterweise liegen die Frequenzwerte, zwischen denen hin und her geschaltet wird, relativ weit auseinander, nämlich im Verhältnis 1:2 bis 1:10, insbesondere 1:2,5 bis 1:5. Es hat sich gezeigt, dass bei derartigen Frequenzabständen die Zellen intensiver beaufschlagt werden als bei Frequenzabständen, die außerhalb dieses Bereiches liegen.

Grundsätzlich stehen dem Fachmann für die Erzeugung der Ultraschallwellen wie auch für die Umschaltung zwischen den unterschiedlichen Frequenzen verschiedene Möglichkeiten zur Verfügung. Erfindungsgemäß wird mit einem Signalgenerator gearbeitet, der zumindest zwei Frequenzen erzeugt, die in Ultraschallwellen mit entsprechenden Frequenzen umgewandelt werden, und dass die Steuereinheit die Umschaltung zwischen den Frequenzen bewirkt. In erster Linie ist hierbei an eine Umschaltung zwischen den Frequenzen der elektrischen Signale des Signalgenerators gedacht. Es liegt aber auch im Rahmen der Erfindung, zusätzlich zwischen den Frequenzen der Ultraschallwellen umzuschalten.

Vorteilhafterweise werden bei dem erfindungsgemäßen Verfahren Frequenzen *f*₁ und *f*₂ vorgegeben, die in einem nicht ganzzahligen Verhältnis zueinander stehen. Entsprechend ist der zuvor genannte Signalgenerator vorzugsweise derart ausgebildet, dass Frequenzen *f*₁ und *f*₂ zur Erzeugung durch den Signalgenerator vorgebbar sind, die in einem nicht ganzzahligen Verhältnis stehen. Die Möglichkeit, Frequenzen in nicht ganzzahligen Verhältnissen vorzugeben, ergibt mehrere Vorteile:
Typischerweise werden die Ultraschallwellen derart erzeugt, dass der Signalgenerator ein elektrisches Signal der vorgegebenen Frequenz erzeugt und dieses Signal in eine Umwandlungseinheit zur Erzeugung von Ultraschallwellen aus dem elektrischen Signal eingespeist wird, typischerweise ein Ultraschallkopf. Solche Umwandlungseinheiten weisen typischerweise mehrere schwingende Elemente auf, beispielsweise ein Piezoelement, auf welches eine zusätzliche Schicht aufgebracht ist. Der Wirkungsgrad solch einer Umwandlungseinheit, d. h. das Verhältnis der in die Umwandlungseinheit eingebrachten elektrischen Leistung zu der akustischen Leistung der ausgehenden Ultraschallwelle ist von mehreren Faktoren abhängig, wie beispielsweise den Materialkonstanten der für die schwingenden Elemente verwendeten Materialien.

Daher liegt typischerweise für eine vorgegebene Wunschfrequenz der Ultraschallwelle eine optimale Frequenz mit einer geringfügigen Abweichung zu dieser Wunschfrequenz, welche einen optimierten Wirkungsgrad aufweist.

So konnte von der Anmelderin gezeigt werden, dass für einen speziellen Ultraschallkopf zur Erzeugung von Ultraschallfrequenzen im Bereich von 3 MHz einerseits und 10 MHz andererseits die Beaufschlagung mit geringfügig abweichenden Frequenzen (3,128 MHz und 9,752 MHz) zu einer erheblichen Erhöhung des Wirkungsgrades für die jeweils erzeugte Ultraschallfrequenz führt. Hierdurch wird die akustische Ausgangsleistung erhöht bzw. es wird eine geringere elektrische Eingangsleistung zur Erzielung einer gewünschten akustischen Ausgangsleistung benötigt, so dass eine geringere Beanspruchung der verwendeten Komponenten und eine geringere Dimensionierung hinsichtlich der elektrischen Leistung des Signalgenerators möglich sind.

Die für eine vorgegebene Wunschfrequenz optimierte Frequenz ist jedoch typischerweise für jeden Ultraschallkopf anders zu wählen, da der Wirkungsgrad auch von Verfahrensparametern wie beispielsweise Temperatur und Diffusionsvorgang beim Verkleben mehrerer Schichten abhängt, so dass im Ergebnis zwei verschiedene Ultraschallköpfe in der Regel unterschiedliche Abhängigkeiten des Wirkungsgrades und daher auch unterschiedliche optimale Frequenzen aufweisen.

Daher ist das erfindungsgemäße System zur Erzeugung von Ultraschallwellen vorteilhafterweise derart ausgeführt, dass mittels eines elektrischen Anschlusses wahlweise mindestens einer von mehreren Ultraschallköpfen anschließbar ist und dass für den jeweiligen Ultraschallkopf jeweils individuelle Frequenzen *f*₁ und *f*₂ vorgebbar sind. Hierdurch ist es möglich, für jeden Ultraschallkopf die wie zuvor beschrieben, optimalen Frequenzen für jeweils vorgegebene Wunschfrequenzen zu bestimmen und die Ultraschallköpfe mit den jeweils individuell optimierten Frequenzen zu betreiben.

Erfindungsgemäß weist der Ultraschallkopf oder der elektrische Anschluss des Ultraschallkopfes ein Speicherelement auf, in dem die für diesen Ultraschallkopf optimierten Frequenzen abgespeichert sind und entsprechend weist das erfindungsgemäße System eine Ausleseeinheit auf, zum Auslesen der abgespeicherten optimalen Frequenzen und Weitergabe an den Signalgenerator.

Vorteilhafterweise ist der Signalgenerator derart ausgeführt, dass Frequenzen mit einer Genauigkeit von 1 Hz vorgebbar sind, so dass eine feine Abstimmung hinsichtlich der optimalen Frequenz möglich ist.

Vorteilhafterweise umfasst der Signalgenerator einen Oszillator und eine Frequenz-Modulationseinheit aufweisen, die aus der Oszillatorfrequenz zumindest zwei verschiedene Frequenzen erzeugt. In diesem Fall braucht der Oszillator nur eine Frequenz erzeugen, aus der die Modulationseinheit dann die gewünschten unterschiedlichen Frequenzen ableitet.

In einer vorzugsweisen Ausführungsform weist der Signalgenerator einen Oszillator, einen Frequenzcontroller und eine Verstärkungseinheit auf. Der Oszillator erzeugt die Grundschwingung, wobei die Frequenz, mit der der Oszillator schwingt, über den mit dem Oszillator verbundenen Frequenzcontroller reguliert wird. Der Ausgang des Oszillators ist mit dem Eingang der Verstärkungseinheit verbunden, so dass am Ausgang der Verstärkungseinheit das Schwingungssignal des Oszillators in verstärkter Form vorliegt. Der Verstärkungsfaktor kann an der Verstärkungseinheit vorgegeben werden. Der Ausgang der Verstärkungseinheit ist mit dem Signalausgang des Signalgenerators verbunden, sodass das verstärkte Oszillatorsignal an den Ultraschallkopf weitergeleitet wird.

In einer weiteren vorzugsweisen Ausführungsform ist die Steuereinheit als Mikrocontroller ausgeführt, das heißt als Kontrolleinheit, welche eine elektronische Steuerung umfasst. Der Mikrocontroller kann beispielsweise durch einen Computer realisiert sein. Über einen Steuerausgang ist der Mikrocontroller mit dem Eingang des Frequenzcontrollers verbunden, sodass der Mikrocontroller letztendlich die Frequenz vorgibt, welche am Signalausgang des Signalgenerators anliegt.

Weiterhin ist es vorteilhaft, wenn die Steuereinheit zusätzlich die Intensität der Ultraschallwelle steuert. Hierzu kann die Steuereinheit zum einen mit einem Steuereingang der Verstärkungseinheit verbunden sein und zum anderen über einen Signaleingang mit einem Signalausgang der Verstärkungseinheit verbunden sein. Über den Signaleingang der Steuereinheit kann somit die tatsächliche Intensität des am Signalausgang des Signalgenerators anliegenden Signals überprüft werden und entsprechend kann der Verstärkungsfaktor korrigiert und entsprechend an den Steuereingang der Verstärkungseinheit weitergeleitet werden, sodass am Signalausgang ein Signal mit einer vorgegebenen Intensität anliegt.

Darüber hinaus ist es vorteilhaft, wenn das System zur Erzeugung von Ultraschallwellen mindestens zwei Ultraschallköpfe mit unterschiedlicher Größe aufweist. Ultraschallköpfe mit unterschiedlicher Größe haben eine unterschiedlich große Auflagefläche zur Auflage auf das zu bestrahlende biologische Gewebe. Je nach Form des Gewebes und abhängig von der gewünschten zu bestrahlenden Fläche kann so ein passender Ultraschallkopf ausgewählt werden. Hierzu ist der Signalausgang des Signalgenerators derart ausgeführt, dass wahlweise ein oder die mindestens zwei Ultraschallköpfe gleichzeitig mit dem Signalausgang verbunden werden können. Dadurch kann entweder ein passender Ultraschallkopf zur Verwendung ausgewählt werden oder es können mehrere Ultraschallköpfe angeschlossen werden, sodass gleichzeitig mehrere Stellen des biologischen Gewebes mit Ultraschallstrahlung beaufschlagt werden können.

In einer weiteren vorteilhaften Ausführungsform weist das System mindestens zwei Ultraschallköpfe auf, welche mit dem Signalausgang des Signalgenerators verbunden werden können, wobei der Signalausgang eine Signalsteuereinheit umfasst, welche automatisch das Signal an jeweils einen der angeschlossenen Ultraschallköpfe weiterleitet. Die Signalsteuereinheit weist einen Speicher zum Abspeichern von Behandlungsprogrammen auf, in denen die Behandlungsdauer und eventuell weitere Parameter wie Stärke und Frequenz der Ultraschallstrahlung oder Umschaltfrequenz für einen bestimmten Ultraschallkopf vorgegeben sind. Die Signalsteuereinheit ist hierfür mit einer Steuereinheit der Ultraschallköpfe verbunden, zur Steuerung der genannten weiteren Parameter. Nach Ablauf der vorgegebenen Behandlungsdauer schaltet die Signalsteuerung automatisch auf den nächsten im Programm angegebenen Ultraschallkopf um und gibt dies durch ein optisches und/oder akustisches Signal an. Weiterhin übermittelt die Signalsteuerung die weiteren Parameter an die Steuereinheit. Die Behandlung kann daraufhin mit dem nun aktiven Ultraschallkopf mit den vorgegebenen weiteren Parametern fortgesetzt werden.

Um die Kombination einer Beschallung des biologischen Gewebes mit Ultraschall mit einer Reizstrombehandlung zu ermöglichen, weist das System in einer weiteren vorteilhaften Ausführungsform einen Reizstromgenerator auf, welcher einen Reizstrom generiert und an einem Reizstromausgang ausgibt. Der Reizstromausgang ist mit einer Kontakteinheit, welche beispielsweise als an sich bekannte Elektrode ausgeführt sein kann, verbunden, sodass über die Kontakteinheit der Reizstrom auf das biologische Gewebe übertragen werden kann. Die Kontakteinheit ist in den Ultraschallkopf integriert, sodass durch Auflegen des Ultraschallkopfes auf das biologische Gewebe sowohl der Reizstrom als auch die Ultraschallstrahlung in das Gewebe eindringen können.

Ebenso ist eine Kombination des erfindungsgemäßen Systems zur Erzeugung von Ultraschallwellen mit einer Vakuumeinheit vorteilhaft. Hierzu weist das erfindungsgemäße System eine Vakuumeinheit mit einem Vakuumanschluss auf, wobei die Vakuumeinheit derart an dem Ultraschallkopf angeordnet ist, dass bei Anlegen des Ultraschallkopfes an das biologische Gewebe ein Unterdruck erzeugt werden kann, welcher das biologische Gewebe an den Ultraschallkopf andrückt. Die Vakuumeinheit kann beispielsweise als Vakuumglocke ausgebildet sein. Diese bildet mit dem Ultraschallkopf eine bauliche Einheit, so dass durch Auflegen des Ultraschallkopfes auf das biologische Gewebe automatisch eine annähernd fluiddichte Verbindung zwischen biologischem Gewebe und Vakuumglocke hergestellt werden kann.

Ebenso ist die Kombination des erfindungsgemäßen Systems zur Erzeugung von Ultraschallwellen mit einer Infraroteinheit zur Wärmebehandlung des biologischen Gewebes vorteilhaft. Hierzu weist das erfindungsgemäße System eine Infraroteinheit zur Erzeugung von Infrarotstrahlung auf, welche eine Infrarotstrahlungsquelle, wie beispielsweise eine Glühröhre, und eine Energieversorgung für Infrarotstrahlungsquelle umfasst. Die Infrarotstrahlungsquelle ist in den Ultraschallkopf integriert und derart ausgerichtet, dass bei Auflegen des Ultraschallkopfs auf das biologische Gewebe die Infrarotstrahlung der Infrarotstrahlungsquelle auf die Oberfläche des biologischen Gewebes einwirkt.

Besonders zweckmäßig ist die Kombination des beschriebenen Systems mit einer Vorrichtung zur Erzeugung von Hochfrequenzströmen, die mit einer Frequenz zwischen 300 kHz bis 10 MHz über zumindest eine nach außen laufende Elektrode auf das Gewebe übertragen werden. Dies kann gleichzeitig oder zeitlich versetzt zur Ultraschallbehandlung erfolgen.

Eine besonders vorteilhafte Anwendung der beschriebenen Ultraschallbehandlung ergibt sich bei Kombination mit der kosmetischen Fettabsaugung. Ursächlich hierfür ist die durch die erfindungsgemäße Ultraschallbehandlung signifikant verbesserte Durchlässigkeit der Zellwände. Dadurch kann von der gleichen Absaugstelle Fett aus wesentlich größerer Entfernung als bisher abgesaugt werden. Gleichzeitig vergleichmäßigt die bessere Durchlässigkeit der Zellen die lokale Fettkonzentration. Einbeulungen oder Wellenbildung an den behandelten Körperpartien werden dadurch ausgeschlossen.

Dabei erfolgt die Ultraschall-Behandlung zweckmäßig einmal direkt vor dem Fettabsaugen und insgesamt drei- bis viermal nach der Fettabsaugung, vorzugsweise täglich oder jeden 2. Tag. Die Behandlungszeit liegt vorzugsweise jeweils zwischen 30 und 60 min.

Weitere Einzelheiten und Merkmale der Erfindung ergeben sich aus den übrigen Ansprüchen und der nachfolgenden Beschreibung eines Ausführungsbeispieles sowie aus der Zeichnung; dabei zeigt
- Figur 1: eine perspektivische Ansicht eines erfindungsgemäßen Ultraschallkopfes und
- Figur 2: ein Blockschaltbild eines erfindungsgemäßen Systems zur Erzeugung von Ultraschallwellen.

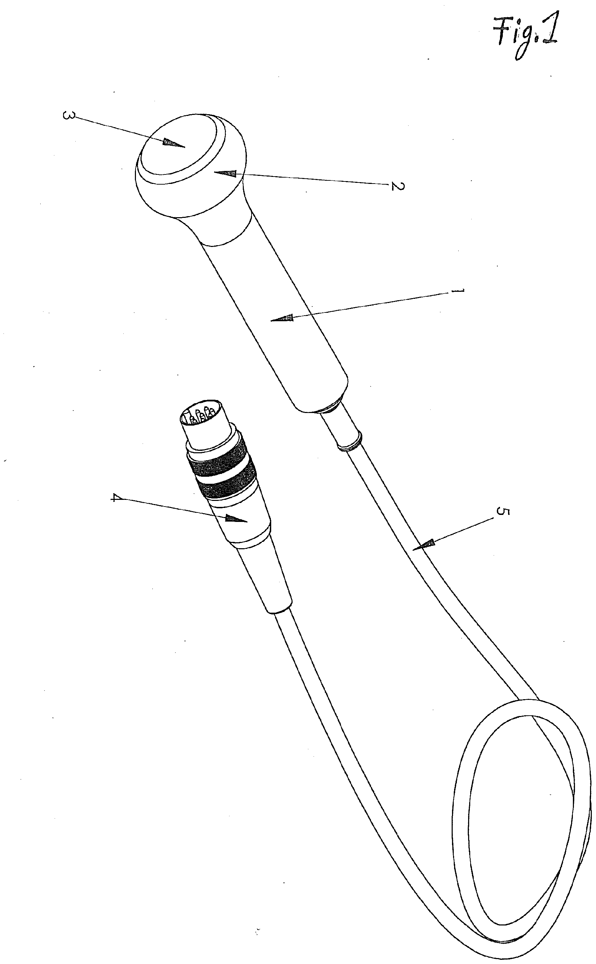

Der in Figur 1 dargestellte Ultraschallkopf umfasst ein Gehäuse 1, welches an dem vorderen Ende ein Kopfteil 2 aufweist, an dem ein Protektor 3 angeordnet ist. An dem Protektor 3 ist eine Auflagefläche ausgebildet, zum Anlegen an das biologische Gewebe. Auf der im Kopfteil 2 liegenden Seite des Protektors 3 ist an dem Protektor ein Piezoelement angebracht, welches die Ultraschallschwingungen erzeugt und an den Protektor 3 weitergibt. In dem Gehäuse 1 ist ferner eine entsprechende Ansteuerelektronik für das Piezoelement enthalten, welche über eine Anschlussleitung 5 und einen Stecker 4 mit einem (nicht dargestellten) Signalgenerator verbunden werden kann. Die in dem Gehäuse 1 enthaltene Ansteuerelektronik wandelt in Verbindung mit dem Piezoelement elektrische Signale, welche über das Kabel 5 übertragen werden, in Ultraschallwellen um, sodass durch Auflegen des Protektors 3 auf biologisches Gewebe dieses mit Ultraschallwellen beaufschlagt werden kann.

Das Gehäuse 1 umfasst ferner eine Kopplungskontrolle 2. Die Kopplungskontrolle registriert, ob der Protektor 3 mit Druck beaufschlagt ist, das heißt ob der Protektor 3 an biologischem Gewebe anliegt. Ist dies der Fall, so gibt die Kopplungskontrolle 2 ein entsprechendes Signal über das Kabel 5 und den Stecker 4 an den (nicht dargestellten) Mikrocontroller des erfindungsgemäßen Systems zur Erzeugung von Ultraschallstrahlung.

In dem Stecker 4 befindet sich ferner ein Speicherbaustein, auf dem Kalibrierungsdaten des Ultraschallkopfes, insbesondere des Piezoelementes gespeichert sind. Der Speicherbaustein kann über in dem Stecker 4 angebrachte Kontakte mit dem Mikrocontroller verbunden werden, sodass der Mikrocontroller die gespeicherten Daten auslesen und abhängig von den Kalibrierungsdaten die Intensität der am Signalausgang des Signalgenerators anliegenden Signale derart wählen, dass eine Ultraschallstrahlung mit einer vorgegebenen Intensität durch das Piezoelement erzeugt wird.

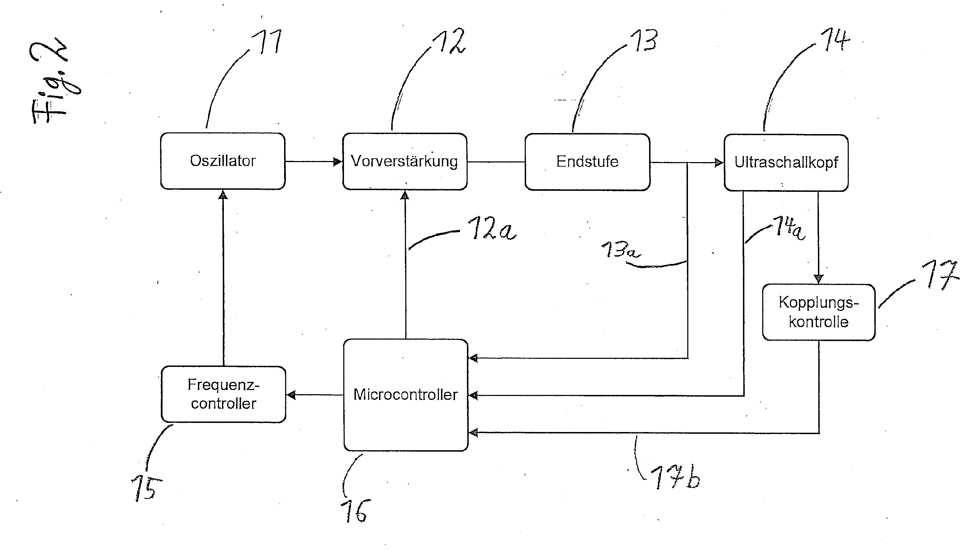

In Figur 2 ist ein Blockschaltbild des erfindungsgemäßen Systems zur Erzeugung von Ultraschallstrahlung dargestellt. Ein Oszillator 11 erzeugt periodische elektrische Signale, die anschließend in einer Verstärkungseinheit verstärkt werden. Diese Verstärkungseinheit besteht aus einer Vorverstärkung 12 und einer Endstufe 13. Das von der Endstufe ausgegebene Signal wird durch den in Figur 1 dargestellten Stecker 4 und das Kabel 5 an den Ultraschallkopf weitergeleitet, so dass im Ultraschallkopf 14 das elektrische Signal in Ultraschallwellen umgewandelt werden kann.

Die Frequenz, mit der der Oszillator 11 schwingt, wird durch einen Frequenzcontroller 15 reguliert. Dieser wird wiederum von einer Steuereinheit 16, welche als Microcontroller ausgeführt ist gesteuert, welcher die aktuell zu erzeugende Frequenz vorgibt. Für den Betrieb sind in dem Mikrocontroller folgende Größen vorgegeben:
- Die gewünschte Intensität der Ultraschallstrahlung,
- die Ultraschallfrequenzen, zwischen denen gewechselt werden soll,
- die Reihenfolge, in der zwischen den vorgegebenen Ultraschallfrequenzen gewechselt werden soll und
- die Umschaltfrequenz, mit der die Ultraschallfrequenzen gemäß der vorgegebenen Reihenfolge gewechselt werden.

Sind beispielsweise drei Ultraschallfrequenzen *f₁* = 1 MHz, *f₂* = 3 MHz und *f₃* = 10 MHz, eine Wechselreihenfolge (1 MHz, 3 MHz, 10 MHz, 3 MHz, 1MHz, etc.), eine Intensität 1 W/cm², und Zeitspannen *T₁* = 1 ms, *T₂* = 5 ms und *T₃* = 15 ms vorgegeben, so gibt der Mikrocontroller zunächst für 0,001 Sekunden das Signal an den Frequenzcontroller 15 zur Erzeugung einer Frequenz mit 1 MHz, danach für 0,005 Sekunden das Signal zur Erzeugung einer Frequenz mit 3 MHz, dann für 0,015 Sekunden das Signal zur Erzeugung einer Frequenz mit 10 MHz und wiederholt diesen Zyklus fortlaufend.

Demgemäß wird ein entsprechendes periodisches Signal mit der gewünschten Frequenz erzeugt und liegt am Ausgang der Endstufe 13 an. Dieser Ausgang ist über eine Leitung 13a mit einem Signaleingang des Mikrocontrollers verbunden, sodass im Mikrocontroller die tatsächlich an der Endstufe anliegende Signalstärke bestimmt werden kann. Der Mikrocontroller gibt nun über die Leitung 12a ein entsprechendes Signal an die Vorverstärkung 12, sodass am Ausgang der Endstufe 13 ein Signal mit der gewünschten Intensität anliegt. Zur Berechnung der gewünschten Intensität des am Ausgang der Endstufe 13 anliegenden elektrischen Signals werden von dem Mikrocontroller über die Leitung 14a die Kalibrierungsdaten des Ultraschallkopfes eingelesen. Mit Hilfe dieser Kalibrierungsdaten berechnet der Mikrocontroller die gewünschte Intensität am Ausgang der Endstufe 13 derart, dass durch den Ultraschallkopf Ultraschallstrahlung mit der vorgegebenen Intensität von 1 W/cm² erzeugt wird.

Darüber hinaus ist der Mikrocontroller über eine Leitung 17b mit der Kopplungskontrolle 7 des Ultraschallkopfes 4 verbunden. Die Kopplungskontrolle gibt bei Anliegen des in Figur 1 dargestellten Protektors 3 an biologischem Gewebe ein Signal an den Mikrocontroller und der Mikrocontroller ist derart ausgeführt, dass er nur bei anliegendem Signal der Kopplungskontrolle ein positives Signal an die Vorverstärkung abgibt. Dies bedeutet, dass bei fehlendem Signal der Kopplungskontrolle, das heißt, wenn der in Figur 1 dargestellte Protektor 3 nicht an dem biologischen Gewebe anliegt, kein positives Signal an die Vorverstärkung abgegeben wird und somit keinerlei Verstärkung stattfindet, sodass am Ausgang der Endstufe 3 allenfalls ein Signal mit geringer Intensität anliegt.

## Patentansprüche

1. System zur Erzeugung von Ultraschallwellen, insbesondere zur kosmetischen und therapeutischen Behandlung, aufweisend einen elektrisch angesteuerten Ultraschallkopf (14), der dazu ausgebildet ist, Ultraschallwellen mit zumindest zwei unterschiedlichen Frequenzen *f₁*, *f₂* zu erzeugen, und eine Steuereinheit (16), die dazu ausgebildet ist, permanent oder zumindest während vorgegebener Zeitspannen zumindest zwischen den zwei vorgegebenen Frequenzwerten *f₁*, *f₂* umzuschalten, wobei das System einen Signalgenerator (11, 12, 13, 15) aufweist, der dazu ausgebildet ist, die zumindest zwei Frequenzen *f₁*, f₂ zu erzeugen, zur Umwandlung in Ultraschallwellen,
wobei der Ultraschallkopf oder ein elektrischer Anschluss des Ultraschallkopfes ein Speicherelement aufweist,
**dadurch gekennzeichnet, dass** die Steuereinheit (16) dazu ausgebildet ist, nach Umschalten auf die Frequenz *f₁* Ultraschallwellen mit dieser Frequenz für eine vorgegebene Zeitspanne *T₁* zu erzeugen und nach Umschalten auf die Frequenz *f₂* Ultraschallwellen mit dieser Frequenz für eine zu *T₁* unterschiedliche vorgegebene Zeitspanne *T*₂ zu erzeugen, wobei die der höheren Frequenz zugeordnete Zeitspanne größer ist als die der niedrigeren Frequenz zugeordnete Zeitspanne, wobei die Zeitspannen *T₁* und *T*₂ im Bereich 0,5 ms bis 50 ms liegen, dass in dem Speicherelement für den Ultraschallkopf optimierte Frequenzen *f₁*, *f₂* abgespeichert sind und dass das System eine Ausleseeinheit aufweist, zum Auslesen der Frequenzen *f₁*, *f₂* und Weitergabe an den Signalgenerator.

2. System zur Erzeugung von Ultraschallwellen nach Anspruch 1,
**dadurch gekennzeichnet,**
**dass** die Zeitspannen *T₁* und *T₂* im Bereich 1 ms bis 20 ms liegen.

3. System zur Erzeugung von Ultraschallwellen nach Anspruch 1,
**dadurch gekennzeichnet,**
**dass** die Zeitspannen *T₁* und *T₂* ein Verhältnis *T₁*/*T₂* aufweisen, das in etwa 3/7 oder in etwa 1/3 oder in etwa 1/4 beträgt.

4. System zur Erzeugung von Ultraschallwellen nach Anspruch 1,
**dadurch gekennzeichnet,**
**dass** die Zeitspannen *T₁* und *T₂* mit *T₁* = 1 ms und *T₂* = 4 ms oder mit *T₁* = 3 ms und *T₂* = 7 ms oder mit *T₁* = 5 ms und *T₂* = 15 ms vorgegeben sind.

5. System zur Erzeugung von Ultraschallwellen nach Anspruch 1,
**dadurch gekennzeichnet,**
**dass** die Ultraschallwellen Frequenzen im Bereich von 20 kHz bis 20 MHz, insbesondere von 0,5 MHz bis etwa 10 MHz, vorzugsweise 0,7 MHz bis etwa 10 MHz aufweisen.

6. System zur Erzeugung von Ultraschallwellen nach Anspruch 1,
**dadurch gekennzeichnet,**
**dass** das Umschalten zwischen Ultraschallwellen erfolgt, deren Frequenzen etwa im Verhältnis 1:2 bis 1:10, insbesondere 1:2,5 bis 1:5, vorzugsweise etwa 1:3 zueinander stehen.

7. System zur Erzeugung von Ultraschallwellen nach einem der vorangegangenen Ansprüche,
**dadurch gekennzeichnet,**
**dass** die von dem Signalgenerator erzeugten Frequenzen *f₁* und *f₂* in einem nicht ganzzahligen Verhältnis vorgebbar sind, insbesondere, dass die Frequenz *f₁* eine hinsichtlich des Wirkungsgrades zwischen elektrischer Eingangsleistung und akustischer Ausgangsleistung des Ultraschallkopfes optimierte Frequenz ist und ebenso die Frequenz *f₂*.

8. System zur Erzeugung von Ultraschallwellen nach einem der vorangegangenen Ansprüche,
**dadurch gekennzeichnet,**
**dass** das System mindestens einen elektrischen Anschluss zum wahlweise Anschließen mindestens eines von mehreren Ultraschallköpfen aufweist und dass für den jeweils angeschlossenen Ultraschallkopf individuelle Frequenzen *f₁* und *f₂* vorgebbar sind.

9. System nach Anspruch 8,
**dadurch gekennzeichnet,**
**dass** jeder der Ultraschallköpfe oder deren elektrischer Anschluss jeweils ein Speicherelement zum Abspeichern von optimalen Frequenzen aufweist.

10. System zur Erzeugung von Ultraschallwellen nach einem der vorangegangenen Ansprüche,
**dadurch gekennzeichnet,**
**dass** der Signalgenerator einen Oszillator und eine Frequenzmodulations-Einheit umfasst, die aus der Oszillator-Frequenz zumindest zwei verschiedene Frequenzen erzeugt.

11. System zur Erzeugung von Ultraschallwellen nach einem der Ansprüche 1-9,
**dadurch gekennzeichnet,**
**dass** der Signalgenerator einen Oszillator (11), einen Frequenzcontroller (15) und eine Verstärkungseinheit (12, 13) umfasst,
wobei der Frequenzcontroller (15) mit dem Oszillator (11) verbunden und derart ausgeführt ist, dass er für eine vorgegebene Frequenz derart auf den Oszillator (11) einwirkt, dass dieser mit der vorgegebenen Frequenz schwingt und
wobei ein Ausgang des Oszillators (11) mit einem Eingang der Verstärkungseinheit (12, 13) verbunden und die Verstärkungseinheit derart ausgeführt ist, dass sie das am Eingang anliegende Signal in etwa um einen vorgegebenen Faktor verstärkt und dass ein Ausgang der Verstärkungseinheit (12, 13) der Signalausgang des Signalgenerators ist.

12. System zur Erzeugung von Ultraschallwellen nach Anspruch 11,
**dadurch gekennzeichnet,**
**dass** die Steuereinheit (16) als Microcontroller mit einem Steuerausgang ausgeführt ist, welcher mit einem Eingang des Frequenzcontrollers (15) verbunden ist, zur Steuerung der durch den Signalgenerator erzeugten Frequenz.

13. System zur Erzeugung von Ultraschallwellen nach Anspruch 12,
**dadurch gekennzeichnet,**
**dass** die Steuereinheit (16) mit einem Steuereingang der Verstärkungseinheit (12, 13) verbunden ist,
**dass** die Steuereinheit (16) einen Signaleingang aufweist, welcher mit dem Signalausgang der Verstärkungseinheit (12, 13) verbunden ist und dass die Steuereinheit (16) derart ausgeführt ist, dass sie abhängig von der Signalstärke des am Signaleingang der Steuereinheit (16) anliegenden Signals derart über den Steuereingang der Verstärkungseinheit (12, 13) auf diese einwirkt, dass die Verstärkungseinheit (12, 13) ein periodisches elektrisches Signal mit einer durch die Steuereinheit (16) vorgegebenen Intensität erzeugt.

14. System zur Erzeugung von Ultraschallwellen nach einem der vorangegangenen Ansprüche,
**dadurch gekennzeichnet,**
**dass** das System mindestens zwei Ultraschallköpfe (14) insbesondere mit unterschiedlicher Größe aufweist und
**dass** der Signalausgang des Signalgenerators derart ausgeführt ist, dass wahlweise ein oder die mindestens zwei Ultraschallköpfe (14) gleichzeitig mit dem Signalausgang des Signalgenerators verbunden werden können.

## Claims

1. System for generating ultrasonic waves, especially for cosmetic and therapeutic treatment, having an electrically actuated ultrasound head (14) which is designed to generate ultrasonic waves at at least two different frequencies *f₁*, *f₂*, and a control unit (16) which is designed, permanently or at least during specified time periods, to switch over at least between the two specified frequency values *f₁*, *f₂*, wherein the system has a signal generator (11, 12, 13, 15) which is designed to generate the at least two frequencies *f₁*, *f₂* for conversion into ultrasonic waves, wherein the ultrasound head or an electrical connection of the ultrasound head has a memory element, **characterised in that**
the control unit (16) is designed, after switching over to the frequency *f₁*, to generate ultrasonic waves at that frequency for a specified time period *T₁* and, after switching over to the frequency *f₂*, to generate ultrasonic waves at that frequency for a specified time period *T*₂ different from *T₁*, wherein the time period assigned to the higher frequency is greater than the time period assigned to the lower frequency, the time periods *T₁* and *T*₂ being in the range of from 0.5 ms to 50 ms;
frequencies *f₁*, *f₂* optimised for the ultrasound head are stored in the memory element; and
the system has a read-out unit for read-out of the frequencies *f₁*, *f₂* and transmission to the signal generator.

2. System for generating ultrasonic waves according to claim 1,
**characterised in that**
the time periods *T₁* and *T*₂ are in the range of from 1 ms to 20 ms.

3. System for generating ultrasonic waves according to claim 1,
**characterised in that**
the time periods *T₁* and *T*₂ have a ratio *T₁*/*T₂* of approximately 3/7 or approximately 1/3 or approximately 1/4.

4. System for generating ultrasonic waves according to claim 1,
**characterised in that**
the time periods *T₁* and *T*₂ specified are *T₁* = 1 ms and *T*₂ = 4 ms or *T₁* = 3 ms and *T*₂ = 7 ms or *T₁* = 5 ms and *T*₂ = 15 ms.

5. System for generating ultrasonic waves according to claim 1,
**characterised in that**
the ultrasonic waves have frequencies in the range of from 20 kHz to 20 MHz, especially from 0.5 MHz to approximately 10 MHz, preferably from 0.7 MHz to approximately 10 MHz.

6. System for generating ultrasonic waves according to claim 1,
**characterised in that**
the switchover takes place between ultrasonic waves the frequencies of which are approximately in a ratio to one another of from 1:2 to 1:10, especially from 1:2.5 to 1:5, preferably approximately 1:3.

7. System for generating ultrasonic waves according to any one of the preceding claims,
**characterised in that**
the frequencies *f₁* and *f₂* generated by the signal generator are specifiable in a non-integer ratio; especially, the frequency *f₁* is a frequency optimised in respect of the degree of efficiency between electrical input power and acoustic output power of the ultrasound head, and likewise the frequency *f₂*.

8. System for generating ultrasonic waves according to any one of the preceding claims,
**characterised in that**
the system has at least one electrical connection for selective connection of at least one of a plurality of ultrasound heads; and individual frequencies *f₁* and *f₂* are specifiable for each connected ultrasound head.

9. System according to claim 8,
**characterised in that**
each of the ultrasound heads or the electrical connection thereof has a respective memory element for storing optimal frequencies.

10. System for generating ultrasonic waves according to any one of the preceding claims,
**characterised in that**
the signal generator comprises an oscillator and a frequency modulation unit which generates at least two different frequencies from the oscillator frequency.

11. System for generating ultrasonic waves according to any one of claims 1 to 9,
**characterised in that**
the signal generator comprises an oscillator (11), a frequency controller (15) and an amplification unit (12, 13),
wherein the frequency controller (15) is connected to the oscillator (11) and is configured in such a way that for a specified frequency it acts on the oscillator (11) in such a way that the latter oscillates at the specified frequency and
wherein an output of the oscillator (11) is connected to an input of the amplification unit (12, 13) and the amplification unit is configured in such a way that it amplifies the signal present at the input approximately by a specified factor; and
an output of the amplification unit (12, 13) is the signal output of the signal generator.

12. System for generating ultrasonic waves according to claim 11,
**characterised in that**
the control unit (16) is configured as a microcontroller having a control output which is connected to an input of the frequency controller (15) to control the frequency generated by the signal generator.

13. System for generating ultrasonic waves according to claim 12,
**characterised in that**
the control unit (16) is connected to a control input of the amplification unit (12, 13);
the control unit (16) has a signal input which is connected to the signal output of the amplification unit (12, 13); and
the control unit (16) is configured in such a way that, in dependence upon the signal strength of the signal present at the signal input of the control unit (16), it acts on the amplification unit (12, 13) via the control input thereof in such a way that the amplification unit (12, 13) generates a periodic electrical signal having an intensity specified by the control unit (16).

14. System for generating ultrasonic waves according to any one of the preceding claims,
**characterised in that**
the system has at least two ultrasound heads (14), especially of different sizes; and
the signal output of the signal generator is configured in such a way that selectively one ultrasound head or the at least two ultrasound heads (14) simultaneously can be connected to the signal output of the signal generator.

## Revendications

1. Système de génération d'ondes ultrasonores, en particulier pour un traitement cosmétique ou thérapeutique, présentant une tête à ultrasons (14) à pilotage électrique qui est réalisée pour générer des ondes ultrasonores ayant au moins deux fréquences f₁, f₂ différentes, et une unité de commande (16) qui est réalisée pour commuter de manière permanente ou du moins pendant des laps de temps prédéfinis au moins entre les deux valeurs de fréquence f₁, f₂ prédéfinies,
le système présentant un générateur de signaux (11, 12, 13, 15) qui est réalisé pour générer les au moins deux fréquences f₁, f₂ pour la conversion en ondes ultrasonores,
la tête à ultrasons ou un raccordement électrique de la tête à ultrasons présentant un élément de mémoire,
**caractérisé en ce que** l'unité de commande (16) est réalisée, après la commutation sur la fréquence f₁, pour générer des ondes ultrasonores ayant cette fréquence pendant un laps de temps T₁ prédéfini, et après la commutation sur la fréquence f₂, pour générer des ondes ultrasonores ayant cette fréquence pendant un laps de temps T₂ prédéfini, différent de T₁,
le laps de temps associé à la fréquence supérieure étant plus long que le laps de temps associé à la fréquence inférieure, les laps de temps T₁ et T₂ étant compris dans la plage de 0,5 ms à 50 ms,
**en ce que** des fréquences f₁, f₂ optimisées pour la tête à ultrasons sont mémorisées dans l'élément de mémoire, et
**en ce que** le système présente une unité de lecture pour lire les fréquences f₁, f₂ et les transmettre au générateur de signaux.

2. Système de génération d'ondes ultrasonores selon la revendication 1, **caractérisé en ce que** les laps de temps T₁ et T₂ sont compris dans la plage de 1 ms à 20 ms.

3. Système de génération d'ondes ultrasonores selon la revendication 1, **caractérisé en ce que** les laps de temps T₁ et T₂ présentent un rapport T₁/T₂ qui correspond approximativement à 3/7 ou approximativement à 1/3 ou approximativement à 1/4.

4. Système de génération d'ondes ultrasonores selon la revendication 1, **caractérisé en ce que** les laps de temps T₁ et T₂ sont prédéfinis par T₁ = 1 ms et T₂ = 4 ms, ou par T₁ = 3 ms et T₂ = 7 ms, ou par T₁ = 5 ms et T₂ = 15 ms.

5. Système de génération d'ondes ultrasonores selon la revendication 1, **caractérisé en ce que** les ondes ultrasonores présentent des fréquences dans la plage de 20 kHz à 20 MHz, en particulier de 0,5 MHz à environ 10 MHz, de préférence de 0,7 MHz à environ 10 MHz.

6. Système de génération d'ondes ultrasonores selon la revendication 1, **caractérisé en ce que** la commutation a lieu entre les ondes ultrasonores dont les fréquences présentent approximativement un rapport mutuel de 1:2 à 1:10, en particulier de 1:2,5 à 1:5, de préférence d'environ 1:3.

7. Système de génération d'ondes ultrasonores selon l'une quelconque des revendications précédentes, **caractérisé en ce que** les fréquences f₁ et f₂ générées par le générateur de signaux peuvent être prédéfinies dans un rapport non entier, en particulier **en ce que** la fréquence f₁ est une fréquence optimisée quant au rendement entre la puissance d'entrée électrique et la puissance de sortie acoustique de la tête à ultrasons, et de même pour la fréquence f₂.

8. Système de génération d'ondes ultrasonores selon l'une quelconque des revendications précédentes, **caractérisé en ce que** le système présente au moins un raccordement électrique pour raccorder au choix au moins l'une de plusieurs têtes à ultrasons, et **en ce que** pour la tête à ultrasons respectivement raccordée, des fréquences f₁ et f₂ individuelles peuvent être prédéfinies.

9. Système selon la revendication 8, **caractérisé en ce que** chacune des têtes à ultrasons ou leur raccordement électrique présente respectivement un élément de mémoire pour mémoriser des fréquences optimales.

10. Système de génération d'ondes ultrasonores selon l'une quelconque des revendications précédentes, **caractérisé en ce que** le générateur de signaux comprend un oscillateur et une unité de modulation de fréquence qui génère au moins deux fréquences différentes à partir de la fréquence d'oscillateur.

11. Système de génération d'ondes ultrasonores selon l'une quelconque des revendications 1 à 9, **caractérisé**
**en ce que** le générateur de signaux comprend un oscillateur (11), un contrôleur de fréquence (15) et une unité d'amplification (12, 13),
le contrôleur de fréquence (15) étant relié à l'oscillateur (11) et réalisé de telle sorte qu'il agit pour une fréquence prédéfinie sur l'oscillateur (11) de telle sorte que celui-ci oscille à la fréquence prédéfinie, et
dans lequel une sortie de l'oscillateur (11) est reliée à une entrée de l'unité d'amplification (12, 13), et l'unité d'amplification est réalisée de telle sorte qu'elle amplifie le signal présent à l'entrée approximativement d'un facteur prédéfini, et
**en ce qu'**une sortie de l'unité d'amplification (12, 13) est la sortie de signal du générateur de signaux.

12. Système de génération d'ondes ultrasonores selon la revendication 11, **caractérisé en ce que** l'unité de commande (16) est réalisée sous la forme d'un microcontrôleur doté d'une sortie de commande qui est reliée à une entrée du contrôleur de fréquence (15) pour commander la fréquence générée par le générateur de signaux.

13. Système de génération d'ondes ultrasonores selon la revendication 12, **caractérisé**
**en ce que** l'unité de commande (16) est reliée à une entrée de commande de l'unité d'amplification (12, 13),
**en ce que** l'unité de commande (16) présente une entrée de signal qui est reliée à la sortie de signal de l'unité d'amplification (12, 13), et
**en ce que** l'unité de commande (16) est réalisée de telle sorte qu'elle agit en fonction de l'intensité de signal du signal présent à l'entrée de signal de l'unité de commande (16) sur l'unité d'amplification (12, 13) par l'intermédiaire de l'entrée de commande de celle-ci de telle sorte que l'unité d'amplification (12, 13) génère un signal électrique périodique d'une intensité prédéfinie par l'unité de commande (16).

14. Système de génération d'ondes ultrasonores selon l'une quelconque des revendications précédentes, **caractérisé**
**en ce que** le système présente au moins deux têtes à ultrasons (14) en particulier de taille différente, et
**en ce que** la sortie de signal du générateur de signaux est réalisée de telle sorte qu'au choix l'une des têtes à ultrasons (14) peut être reliée à la sortie de signal du générateur de signaux ou les au moins deux têtes à ultrasons peuvent y être reliées en même temps.
